# EUROPÄISCHE PATENTSCHRIFT

(11) **EP 2 337 841 B1**
(45) Veröffentlichungstag und Bekanntmachung des Hinweises auf die Patenterteilung: **07.11.2012**
(21) Anmeldenummer: 09778365.8
(22) Anmeldetag: 07.09.2009
(51) Int. Cl.: C12M 1/34, C12M 1/42, C12M 3/00

(54) **ELEKTRODENEINRICHTUNG, GENERATOREINRICHTUNG UND VERFAHREN ZUR STROMERZEUGUNG DURCH MEMBRANPOTENTIAL-ABLEITUNG**
ELECTRODE DEVICE, GENERATOR DEVICE AND METHOD FOR POWER GENERATION BY MEANS OF MEMBRANE-POTENTIAL SHUNTING
DISPOSITIF D'ÉLECTRODES, ENSEMBLE GÉNÉRATEUR ET PROCÉDÉ POUR PRODUIRE DE L'ÉNERGIE AU MOYEN D'UNE DÉRIVATION DE POTENTIEL DE MEMBRANE

(30) Priorität: 16.09.2008 DE 102008047399
(43) Veröffentlichungstag der Anmeldung: 29.06.2011
(73) Patentinhaber: Max-Planck-Gesellschaft zur Förderung der Wissenschaften e.V., 80539 München (DE)
(72) Erfinder: SPATZ, Joachim, 70569 Stuttgart (DE); RUSTOM, AMIN, 69469 Weinheim/Oberpfalz (DE)
(74) Vertreter: Hertz, Oliver
(86) Internationale Anmeldenummer: PCT/EP2009/006469
(87) Internationale Veröffentlichungsnummer: WO 2010/031506

(56) Entgegenhaltungen:
- EP-A2- 1 344 551
- DE-A1- 10 108 968
- DE-A1- 19 827 957
- DE-A1-102005 030 859
- US-A- 4 969 468
- US-A1- 2002 028 991
- US-A1- 2004 063 100
- GRISS P ET AL: "Spiked biopotential electrodes", 20000123; 20000123 - 20000127, 23. Januar 2000 (2000-01-23), Seiten 323-328, XP010377147,
- METZ S ET AL: "Microelectrodes with three-dimensional structures for improved neural interfacing", PROCEEDINGS OF THE 23RD. ANNUAL INTERNATIONAL CONFERENCE OF THE IEEE ENGINEERING IN MEDICINE AND BIOLOGY SOCIETY. 2001 CONFERENCE PROCEEDINGS. (EMBS). INSTANBUL, TURKEY, OCT. 25 - 28, 2001; [ANNUAL INTERNATIONAL CONFERENCE OF THE IEEE ENGINEERING IN, Bd. 1, 25. Oktober 2001 (2001-10-25), Seiten 765-768, XP010593487, ISBN: 978-0-7803-7211-5

## Beschreibung

Die Erfindung betrifft eine Elektrodeneinrichtung, die zur Ableitung eines Membranpotentials an mindestens einer Membran-umhüllten Zelle, insbesondere an einer Vielzahl von Zellen eingerichtet ist. Des Weiteren betrifft die Erfindung eine Generatoreinrichtung, die mit der Elektrodeneinrichtung ausgestattet und zur Stromerzeugung durch Ableitung eines Membranpotentials eingerichtet ist. Schließlich betrifft die Erfindung auch ein Verfahren zur Stromerzeugung durch Ableitung eines Membranpotentials an mindestens einer biologischen oder synthetischen Zelle. Anwendungen der Erfindung bestehen bei der Erzeugung elektrischen Stroms zum Betrieb eines elektrischen Geräts und/oder zum Aufladen eines Akkumulators.

Die Bereitstellung elektrischer Energie stellt eine der größten Herausforderungen der heutigen technischen Entwicklung dar. Bei der Entwicklung von Stromgeneratoren ist die Ablösung der herkömmlichen Energieumwandlung aus fossilen Brennstoffen durch nachhaltige, erneuerbare Energiequellen von besonderem Interesse. Neben Energiequellen auf der Grundlage von zum Beispiel Wasser- oder Windkraft, Solarenergie oder geothermischer Energie zählen auch Energiequellen auf der Grundlage biologischer Materialien zu den erneuerbaren Energiequellen.

Das Hauptkonzept zur Energieumwandlung unter Verwendung biologischer Materialien basiert auf der Erzeugung und Verbrennung von Biogas (hauptsächlich Methan) oder von Wasserstoff (siehe J. Niessen et al. in "Letters in Applied Microbiology", Band 41, 2005, S. 286-290). Die Energieumwandlung aus Biogas ist wegen des bei der Verbrennung entstehenden Kohlendioxids und wegen des Auftretens von Nebenprodukten, wie zum Beispiel Ammoniak und Schwefelwasserstoff, die aufwändige Reinigungsschritte erfordern, nachteilig. Weitere Konzepte basieren auf so genannten Biobrennstoff-Zellen, die auf der Grundlage eines enzymatischen Abbaus von Makromolekülen, wie zum Beispiel Zucker, betrieben werden (siehe R. F. Service in "Science", Band 296, 2002, S. 1223). Auch diese Konzepte haben keine Routineanwendung gefunden, da sich die Energieausbeute, die beschränkte Stabilität der Energieumwandlung und die hohen Kosten als nachteilig erwiesen haben.

Aus der Zellbiologie ist allgemein bekannt, dass eukariotische biologische Zellen über die Zellmembran ein Membranpotential bilden. Die Zellmembranen enthalten Membranteile, die für einen Ionentransport ausgelegt sind. Diese Membranteile, die Ionenpumpen und Ionenkanäle umfassen, werden im folgenden kurz als Ionenpumpen bezeichnet. Durch den Ionentransport wird über die Zellmembran ein elektrochemischer Gradient erzeugt, der das Membranpotential bildet. Das Membranpotential kann mit elektrophysiologischen Verfahren gemessen werden (siehe zum Beispiel E. Neher in "Science", Band 256, 1992, S. 498-502; W. Baumgartner et al. in "Biophysical Journal", Band 77, 1999, S. 1980-1991; DE 101 08 968 A1; oder DE 198 27 957 A1).

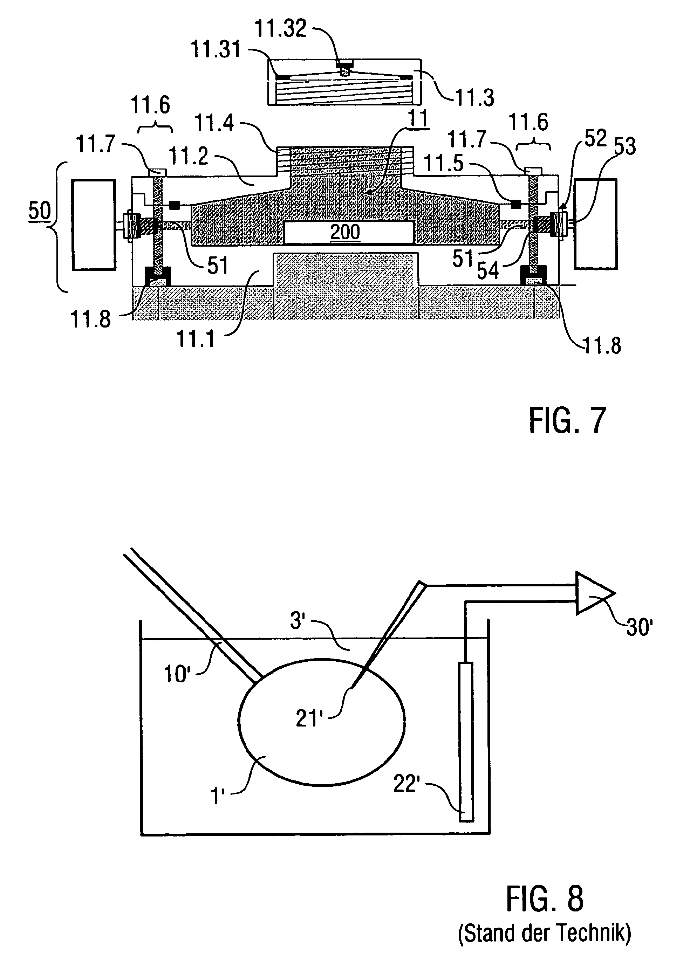

Die herkömmliche elektrophysiologische Messung des Membranpotentials ist schematisch in Figur 8 illustriert. Eine biologische Zelle 1' wird mit einer Pipette 10' in einem Kultivierungsmedium 3' gehalten. Eine Anode 22' ist in das Kultivierungsmedium 3' eingetaucht und eine Kathode 21' ragt vom Kultivierungsmedium 3' isoliert in das Innere der Zelle 1'. Die Anode 22' und die Kathode 21' sind mit einem Verstärker 30' verbunden, mit dem elektrische Signale, welche das abgeleitete Membranpotential repräsentieren, verstärkt und für die weitere Verarbeitung vorbereitet werden. Die Pipette 10' bildet eine Zellaufnahme, die, wie zum Beispiel aus der Praxis für die Messung an adhärenten Zellen bekannt ist, durch ein Substrat im Kultivierungsmedium 3' ersetzt sein kann, auf dem die Zelle 1' angeordnet ist.

Die Anwendung der herkömmlichen Messeinrichtung gemäß Figur 8 ist auf die Messung des Membranpotentials an einzelnen Zellen beschränkt. Da als Kathode 21' bisher Glaselektroden oder metallgefüllte Glaskapillaren mit Spitzendurchmessern oberhalb von 100 nm verwendet werden, ergeben sich Probleme durch eine geringe Toleranz der Zelle 1' gegenüber der Kathode 21' und eine Beschränkung auf hohe Messwiderstände. So können zum Beispiel Elektroden mit Durchmessern oberhalb von 10 µm von der Zelle nur für einige Stunden toleriert werden. Der Messwiderstand ist in der Größenordnung von 100 MΩ, wodurch die abgeleiteten Ströme auf minimale Ladungsmengen begrenzt werden.

Eine elektrophysiologische Messung des Membranpotentials mit mehreren Elektroden ist in DE 10 2005 030 859 A1 beschrieben. Eine isolierende Platte wird als Auflage für eine oder mehrere Zellen verwendet. Elektroden mit elektrisch leitenden Oberflächen ragen durch die Platte in die mindestens eine Zelle. Von Nachteil ist, dass durch den mechanischen Kontakt zwischen der mindestens einen Zelle und der Platte ein hoher elektrischer Abdichtwiderstand realisiert werden muss. Dadurch ergeben sich hohe Mindestabstände der Elektroden und beschränkte Signalamplituden.

Die Aufgabe der Erfindung ist es, eine verbesserte Elektrodeneinrichtung bereitzustellen, die zur Membranpotential-Ableitung an Membran-umhüllten Zellen geeignet ist und mit der Nachteile herkömmlicher Techniken überwunden werden können. Die Aufgabe der Erfindung ist es des Weiteren eine verbesserte Generatoreinrichtung zur Erzeugung elektrischen Stroms unter Verwendung biologischer Materialien bereitzustellen, mit der Beschränkungen herkömmlicher Generatoreinrichtungen überwunden werden und die sich insbesondere durch eine erhöhte Lebensdauer, verbesserte Betriebsstabilität und verringerte Kosten auszeichnet. Die Aufgabe der Erfindung ist es auch, ein verbessertes Verfahren zur Energieumwandlung unter Verwendung biologischer Materialien, insbesondere zur Stromerzeugung bereitzustellen, mit dem Nachteile herkömmlicher erneuerbarer Energiequellen auf der Grundlage biologischer Materialien vermieden werden.

Diese Aufgaben werden durch eine Elektrodeneinrichtung, eine Generatoreinrichtung bzw. durch ein Verfahren mit den Merkmalen der unabhängigen Ansprüche gelöst. Vorteilhafte Ausführungsformen und Anwendungen der Erfindung ergeben sich aus den abhängigen Ansprüchen.

Gemäß einem ersten Gesichtspunkt der Erfindung wird die obige Aufgabe durch eine Elektrodeneinrichtung gelöst, die zur Membranpotential-Ableitung an Membran-umhüllten Zellen eingerichtet ist und eine Zellaufnahme zur adhärenten Fixierung der Zellen und einen Elektrodenträger mit mindestens zwei Elektroden einer ersten Polarität aufweist, die als Vorsprünge über einer Oberfläche des Elektrodenträgers gebildet und relativ zu der Oberfläche des Elektrodenträgers elektrisch isoliert sind. Der Elektrodenträger ist erfindungsgemäß so gebildet, dass wenn die Zellaufnahme die Zellen trägt, die Elektroden gleichzeitig in die Zellen ragen.

Gemäß einem zweiten Gesichtspunkt der Erfindung wird die Aufgabe durch eine Generatoreinrichtung gelöst, die zur Erzeugung elektrischen Stroms durch die Ableitung eines Membranpotentials an Zellen eingerichtet ist und eine Elektrodeneinrichtung gemäß dem oben genannten ersten Gesichtspunkt aufweist, wobei auf der Oberfläche der Zellaufnahme mindestens eine Zelle, vorzugsweise jedoch eine Vielzahl von Zellen angeordnet ist.

Gemäß einem dritten Gesichtspunkt wird die Aufgabe durch ein Verfahren zur Erzeugung von elektrischem Strom durch die Ableitung eines Membranpotentials an Zellen gelöst, die auf der Zellaufnahme der Elektrodeneinrichtung gemäß dem oben genannten ersten Gesichtspunkt in einem adhärenten Zustand angeordnet sind, wobei der elektrische Strom mit den Elektroden und mindestens einer Gegenelektrode abgeleitet wird, die mit den Zellen in direktem elektrischen Kontakt (Berührung) oder, z. B. über ein leitendes Medium, in mittelbarem elektrischen Kontakt steht.

Vorteilhafterweise wird durch den Elektrodenträger mit den mindestens zwei Elektroden eine Elektrodenanordnung realisiert, die im Vergleich zu herkömmlichen Techniken eine erhöhte Stabilität der Elektroden und einen vereinfachten Aufbau aufweist. Es wird insbesondere die Bildung der Elektroden mit einem im Vergleich zu herkömmlichen Techniken verminderten Elektrodendurchmesser ermöglicht, wodurch die Toleranz der Zelle gegenüber der Elektrode verbessert und der Messwiderstand verringert werden.

Vor der Erfindung war es nicht bekannt, die Potentialableitung zur Stromerzeugung zu verwenden. Da bekannt war, dass die Ableitung des Membranpotentials selbst bei Zellen mit starkem Ionentransport, wie zum Beispiel von *Xenopus-*Eizellen, nur einen elektrischen Strom von bis zu 100 µA bei 200 mV ergibt (siehe obige Publikation von W. Baumgartner et al.), wurde vor der Erfindung eine Ausnutzung des Membranpotentials zur Energieumwandlung, insbesondere zur Stromerzeugung nicht in Betracht gezogen. Im Gegenteil, es war sogar notwendig, Messsysteme zusätzlich mit Verstärkern auszustatten, ohne die eine elektrophysiologische Messung der extrem geringen Ströme und Spannungen nicht möglich wäre. Dieses Vorurteil wird mit der Erfindung überwunden. Der Elektrodenträger mit mehreren Elektroden ermöglicht, dass die Elektroden gleichzeitig und insbesondere elektrisch parallel geschaltet in direkten elektrischen Kontakt mit dem Inneren von mindestens einer, vorzugsweise jedoch einer Vielzahl von Zellen gebracht werden. Die Erfinder haben erkannt, dass durch die Verwendung des Elektrodenträgers die Membranpotential-Ableitung skalierbar und somit die Beschränkung auf unpraktikabel geringe Ströme überwindbar ist. Des Weiteren ist es möglich, durch eine Parallelschaltung, eine Serienschaltung oder eine Kombination von Parallel- und Serienschaltung die Stromstärke und/oder die Spannung der Generatoreinrichtung in Abhängigkeit von den Bedingungen der konkreten Anwendung einzustellen.

Die Erfindung hat die folgenden weiteren Vorteile. Die erfindungsgemäße Generatoreinrichtung stellt eine erneuerbare Stromquelle dar, die als makroskopische Stromversorgung in Verbraucher-Stromkreisen geeignet ist. Die Kontaktierung der Zellen erfolgt durch Selbstintegration der Zellen an den Elektroden der Elektrodeneinrichtung. Der Elektrodenträger hat eine durch die Elektrodenvorsprünge nano- oder mikrostrukturierte Oberfläche. Durch die Struktur wird die Bindung der Zellen an die Vorsprünge und somit die Aufnahme der Elektroden in die Zellen und deren Kontaktierung gefördert. Weitere Vorteile der Erfindung bestehen in der Umweltverträglichkeit der Generatoreinrichtung und der Nachhaltigkeit sowie Klimaneutralität der Stromerzeugung.

In Abhängigkeit von der Anwendung der Erfindung kann es aber auch ausreichend sein, dass nur eine einzige Elektrode auf dem Elektrodenträger vorgesehen ist und/oder das Membranpotential von einer einzigen Zelle abgeleitet wird. Diese Varianten gehören somit ebenfalls zum Gegenstand der vorliegenden Erfindung.

Der Begriff "Zelle" (oder: Zellelement, Kompartiment) bezieht sich allgemein auf einen von einer Membran eingehüllten Flüssigkeitsbereich, wobei die Membran zur Erzeugung des Membranpotentials zwischen dem Flüssigkeitsbereich im Inneren der Zelle und einer Flüssigkeit, zum Beispiel einem Kultivierungsmedium, in der Umgebung der Zelle eingerichtet ist. Die Membran enthält Proteine, welche Ionenpumpen (und Ionenkanäle) bilden und zum Ionentransport zwischen der äußeren Umgebung der Zelle und dem Innenraum der Zelle eingerichtet sind.

Die erfindungsgemäße Generatoreinrichtung basiert somit auf der Verwendung biologischer Materialien, indem das elektrochemische Membranpotential von kultivierten biologischen Zellen, die naturgemäß Ionenpumpen enthalten, oder von synthetischen Lipid-Vesikeln ("Phantomzellen"), die in die Membran integrierte Ionenpumpen enthalten, welche das Potential zwischen dem Innenraum und der äußeren Umgebung der Vesikeln aufbauen, zur Stromerzeugung verwendet wird.

Biologische Zellen haben den Vorteil, dass die Fähigkeit, ein Membranpotential zu bilden, durch die vorhandenen Ionenpumpen natürlich gegeben ist. Als biologische Zellen können eukariotische Zellen oder prokariotische Zellen, wie z. B. Bakterien, verwendet werden, wobei eukariotische Zellen aufgrund ihrer größeren Dimension bevorzugt werden. Die Ionenpumpen in der Membran der Vesikeln werden mit an sich bekannten biotechnologischen Verfahren, vorzugsweise unter Verwendung genetisch modifizierter biologischer Zellen bereitgestellt. Die Lebensdauer der Zellen kann durch Verwendung immortalisierter Zellen erhöht werden, in denen Proteine kontinuierlich neu gebildet werden, wobei deren Faltung kontrolliert und an variable Bedingungen angepasst wird.

Synthetische Zellen haben den Vorteil, dass die Lebensdauer der Zellen im Vergleich zu lebenden biologischen Zellen erhöht werden kann und weniger strikte Anforderungen an die Umgebungsbedingungen gestellt werden, als dies bei den Kultivierungsbedingungen von biologischen Zellen der Fall ist.

Gemäß einer bevorzugten Ausführungsform der Erfindung sind die Elektroden als Vorsprünge gebildet, die relativ zu der Oberfläche der Zellaufnahme elektrisch isoliert sind. Besonders bevorzugt weist jede der Elektroden einen Isolatormantel auf, der die Elektrode mit Ausnahme von deren freien Ende umgibt. Der Isolatormantel erstreckt sich in Längsrichtung der Elektrode und lässt die Spitze der Elektrode frei.

Gemäß einer bevorzugten Ausführungsform der Erfindung ist mindestens eine Gegenelektrode einer zweiten, relativ zu den genannten Elektroden entgegengesetzten Polarität vorgesehen, wobei die mindestens eine Gegenelektrode auf der Oberfläche des Elektrodenträgers und/oder an einem von dem Elektrodenträger getrennten Gegenelektrodenträger gebildet ist. Die Bereitstellung der mindestens einen Gegenelektrode in einer vorbestimmten Position relativ zu den Elektroden ermöglicht vorteilhafterweise eine Optimierung der gegenseitigen geometrischen Ausrichtung der Elektroden und der mindestens einen Gegenelektrode zur Erzielung eines über die Zellen gleichförmig abgeleiteten Stromfluss.

Die mindestens eine Gegenelektrode kann auf dem Elektrodenträger und/oder dem Gegenelektrodenträger eine einheitliche elektrisch leitende Schicht oder eine Vielzahl von elektrisch leitenden Elektrodenschichtsegmenten umfassen. Die einheitliche elektrisch leitende Schicht hat den Vorteil, dass mit einer einzigen Gegenelektrode der elektrische Kontakt mit einer Vielzahl von Zellen, zum Beispiel mit einer Zell-Monoschicht auf der Oberfläche des Elektrodenträgers hergestellt werden kann. Die elektrisch leitenden Elektrodenschichtsegmente können so konfiguriert sein, dass sie ansteuerbar sind, z. B. einzeln mit einem Verbraucher-Stromkreis verbunden sind. Die einzeln ansteuerbaren Elektrodenschichtsegmente haben Vorteile, wenn auf der Oberfläche der Trägereinrichtung eine nicht geschlossene Schicht der Zellen vorgesehen ist. Des Weiteren können die Elektrodenschichtsegmente Vorteile bei der Vermeidung von Kurzschlüssen zwischen den Elektrodenschichtsegmenten und den Elektroden entgegengesetzter Polarität haben. Alternativ oder zusätzlich kann die Gegenelektrode einen Elektrodenstab umfassen, der in ein Ionen-enthaltendes Medium in der Umgebung der Zellen eingetaucht wird.

Besonders bevorzugt stellt mindestens eines von dem Elektrodenträger und dem Gegenelektrodenträger die Zellaufnahme dar. Erfindungsgemäß können die Zellen, deren Membranpotential abgeleitet wird, unmittelbar auf dem Elektrodenträger und/oder dem Gegenelektrodenträger adhärent fixiert sein.

In Abhängigkeit von der Art der transportierten Ionen kann sich im Innenraum der Zelle ein negatives oder ein positives Potential relativ zur äußeren Umgebung der Zelle bilden. Die Elektroden, die in den Innenraum der Zellen ragen, und die mindestens eine Gegenelektrode, welche in direktem oder indirektem elektrischem Kontakt mit der Außenseite der Zelle steht, haben allgemein relative zueinander entgegen gesetzte Polaritäten. Für ein negatives Potential im Innenraum relativ zur äußeren Umgebung bilden die Elektroden Kathoden, während die mindestens eine Gegenelektrode mindestens eine Anode bildet. Mit einem umgekehrten Potential bildet die Elektrode eine Kathode, während die Gegenelektrode eine Anode bildet.

Gemäß einer besonders bevorzugten Ausführungsform der Erfindung weist der Elektrodenträger einen Mehrschichtaufbau mit einer Elektrodenschicht, einer Isolatorschicht und einer Gegenelektrodenschicht auf, wobei die Elektroden elektrisch isoliert von der Elektrodenschicht durch die Isolatorschicht und die Gegenelektrodenschicht über die Oberfläche der Gegenelektrodenschicht ragen. Vorteilhafterweise wird damit ein besonders kompakter Aufbau des Elektrodenträgers mit allen Elektroden und Gegenelektroden geschaffen.

Vorteilhafterweise kann der Kontakt zwischen den Elektroden und der mindestens einen Gegenelektrode einerseits und den Zellen andererseits verbessert werden, wenn mindestens eine der Elektroden und/oder Gegenelektroden eine Beschichtung trägt, welche die Wechselwirkung zwischen der Elektrodenoberfläche und der Zellmembran verstärkt. Vorteilhafterweise kann durch die Beschichtung zwischen den Zellen und den Elektroden und/oder Gegenelektroden die Leitfähigkeit erhöht und der Übergangswiderstand vermindert werden. Vorzugsweise tragen die auf der Oberfläche des Elektrodenträgers vorragenden Elektroden eine Beschichtung, die eine Endozytose der Membran aktiviert. Die Endozytose-aktivierende Beschichtung hat den Vorteil, dass die Aufnahme der Elektroden in den Innenraum der Zellen erleichtert wird. Beispielsweise bewirkt die Endozytose-aktivierende Beschichtung bei biologischen Zellen, dass diese aufgrund ihrer natürlichen Zellbewegung aktiv die Elektroden aufnehmen, so dass diese den Kontakt im Innenraum der Zellen bilden. Alternativ oder zusätzlich kann die mindestens eine Gegenelektrode eine Adhäsions und/oder Leitfähigkeits-steigernde Beschichtung tragen. Vorteilhafterweise wird damit die adhärente Bindung der Zelle zur Oberfläche des Elektroden- oder Gegenelektrodenträgers verstärkt. Des Weiteren wird ein Verschluss von ggf. auftretenden Löchern in einer Schicht mit einer Vielzahl von Zellen stimuliert.

Die erfindungsgemäße Verwendung des Membranpotentials zur Stromerzeugung kann erleichtert werden, wenn die Elektroden, die in das Innere der Zellen ragen, einen geringeren Durchmesser hat, als dies bei den herkömmlichen elektrophysiologischen Messungen der Fall ist. Vorzugsweise ist der Durchmesser der Elektroden geringer als 10 µm, besonders bevorzugt kleiner als 1 µm, wie zum Beispiel weniger als 500 nm, insbesondere weniger als 100 nm, wie zum Beispiel weniger als 10 nm, bis zum Beispiel 2 nm oder sogar 1 nm. Elektroden mit einem derartig geringen Durchmesser werden vorteilhafterweise leichter von Zellen, insbesondere biologischen Zellen toleriert. Somit wird die Langzeitstabilität der Stromerzeugung verbessert. Des Weiteren kann der Widerstand zwischen jeder der Elektroden und dem Inneren der Zellen vermindert werden. Die Elektroden können insbesondere eine Anordnung von Leitern mit einem Durchmesser im Sub-µm-Bereich (so genannte Nanodrähte) umfassen, die den Abgriff des Spannungspotentials verbessern, der zwischen den Innenräumen und der äußeren Umgebung der Zellen gebildet ist.

Von Vorteil ist ferner, wenn die freie Elektrodenfläche der Elektroden, zum Beispiel eine Spitze der Elektrode, einen Abstand von der Oberfläche des Elektrodenträgers aufweist, der mindestens 10 nm, vorzugsweise mindestens 100 nm, besonders bevorzugt mindestens 1 µm, wie zum Beispiel 50 µm oder mehr, bis in den mm-Bereich beträgt. Dies ermöglicht eine sichere Positionierung der Elektroden im Inneren der Zellen. Es können insbesondere synthetische Zellen mit einem Durchmesser bis in den mm-Bereich sicher kontaktiert werden.

Erfindungsgemäß können zwei oder mehr Elektroden vorgesehen sein. Gemäß bevorzugten Varianten der Erfindung sind z. B. mindestens zehn, insbesondere mindestens einhundert, z. B. mindestens 1000, wie z. B. 10.000 oder mehr Elektroden vorgesehen. Die Elektroden sind vorzugsweise elektrisch miteinander verbunden. Die Elektroden bilden besonders bevorzugt ein 1- oder 2-dimensionales Array von Elektrodenspitzen, die von der Oberfläche des Elektrodenträgers hervorragen und elektrisch von der mindestens einen Gegenelektrode entgegengesetzter Polarität isoliert sind. Vorteilhafterweise kann mit der elektrischen Verbindung der Elektroden bei Bereitstellung einer Vielzahl von Zellen auf dem Elektrodenträger das Membranpotential an allen Zellen gleichzeitig abgeleitet werden.

Gemäß einer besonders bevorzugten Ausführungsform der Erfindung bilden die Zellen, insbesondere biologische Zellen und/oder synthetische Vesikeln, auf der Oberfläche des Elektrodenträgers eine geschlossene Zellschicht. Die Stromableitungen von den Membranpotentialen der einzelnen Zellen werden parallel geschaltet und erhöhen damit vorteilhafterweise den Ausgangsstrom der erfindungsgemäßen Generatoreinrichtung. Vorteilhafterweise kann mit einer Kultur biologischer Zellen auf einer Oberfläche der Trägereinrichtung von 1 cm² eine Gesamtleistung von 7 µW erzeugt werden. Somit ist die Leistungsfähigkeit vergleichbar mit der Leistungsfähigkeit von Solarzellen gleicher Fläche. Im Unterschied zu Solarzellen, die auf dem photovoltaischen Effekt basieren, kann die erfindungsgemäße Generatoreinrichtung jedoch vollständig unabhängig von Licht betrieben werden. Sie erfordert weniger Platz und unterliegt weniger restriktiven Betriebsbedingungen als eine Solarzellen-Batterie.

Vorteilhafterweise bestehen verschiedene Varianten der Verwendung des erfindungsgemäß erzeugten elektrischen Stroms. Gemäß der Erfindung sind die Elektroden und die mindestens eine Gegenelektrode dazu eingerichtet, unmittelbar mit einem Verbraucher-Stromkreis verbunden zu werden. Es kann eine Leitungsverbindung mit dem Verbraucher-Stromkreis vorgesehen sein. Der Verbrauch wirkt sich vorteilhafterweise unmittelbar auf die Ableitung des Membranpotentials und die Aktivität der Ionenpumpen aus. Gemäß einer zweiten Variante kann eine Verbindung mit einer Akkumulatoreinrichtung vorgesehen sein. Die Akkumulatoreinrichtung ist allgemein ein elektrischer Energiespeicher, wie zum Beispiel ein elektrochemischer Akkumulator oder ein Kondensator mit einer vorbestimmten Kapazität, dessen konkrete Ausführung in Abhängigkeit von den Anforderungen der konkreten Anwendung der Generatoreinrichtung gewählt ist. Die Akkumulatoreinrichtung ist mit dem erfindungsgemäß erzeugten Strom aufladbar, wobei sie den Vorteil hat, dass ein Verbraucher-Stromkreis nicht direkt mit den Elektroden verbunden, sondern von diesen entkoppelt ist. Somit können störende Rückwirkungen des Verbraucher-Stromkreises, wie zum Beispiel schnelle Laständerungen auf die mindestens eine Zelle in der Generatoreinrichtung vermieden werden.

Gemäß einem weiteren vorteilhaften Merkmal der erfindungsgemäßen Generatoreinrichtung ist eine Kultivierungseinrichtung vorgesehen. Die Kultivierungseinrichtung ist zur Zuführung eines Ionen-enthaltenden Mediums in die Umgebung der Zellen eingerichtet. Das Ionen-enthaltende Medium ist eine Flüssigkeit (z. B. ein Kultivierungsmedium), mit der die Zellen versorgt und regeneriert werden. Bei Verwendung biologischer Zellen umfasst die Kultivierungseinrichtung ein Reservoir für das Kultivierungsmedium, wie es aus der Zellbiologie bekannt ist, und eine Zufuhreinrichtung zur Zuleitung des Kultivierungsmediums zu den Zellen auf der Zellaufnahme. Mit dem Ionen-enthaltenden Medium werden laufend an der Umgebung der Zellen Ionen bereitgestellt, die unter der Wirkung der Ionenpumpen in das Innere der Zellen transportiert werden und so das Membranpotential aufbauen.

Wenn gemäß einem weiteren vorteilhaften Merkmal der Erfindung die Zellen in ihrem Innenraum mindestens eine Ionen-bindende Substanz enthalten, können sich Vorteile für die Lebensdauer oder Stabilität der Zellen ergeben. Die Ionen-bindende Substanz wirkt als Puffer im Innenraum der Zelle. Die durch die Membran in den Innenraum transportierten Ionen werden von der Ionen-bindenden Substanz gesammelt. Somit werden insbesondere bei biologischen Zellen die Lebensdauer der Generatoreinrichtungen erhöht. Mit der Ionen-bindenden Substanz wird eine Vergiftung der Zellen unterdrückt oder vollständig vermieden. Die Ionen-bindende Substanz (Ionenfänger) umfasst zum BeispielEthylendiamintetraessigsäure (EDTA), Ascorbinsäure (Vitamin C) oder Glutathion.

Bevorzugte Anwendungen der erfindungsgemäßen Generatoreinrichtung bestehen bei der Stromversorgung von elektrischen Geräten mit einer relativ geringen elektrischen Leistung, wie Körperimplantaten, zum Beispiel Herzschrittmacher oder Hörgeräte, wobei jedoch durch die Skalierbarkeit der Generatoreinrichtung auch Geräte mit erhöhtem Stromverbrauch, wie Haushaltsgeräte versorgt werden können. Für die Versorgung von Körperimplantaten ist von besonderem Vorteil, dass die Generatoreinrichtung unmittelbar mit Nährstoffen des Körpers, insbesondere aus dem an ein Implantat angrenzenden Gewebe versorgt werden kann.

Weitere Einzelheiten und Vorteile der Erfindung werden im Folgenden unter Bezug auf die beigefügten Zeichnungen beschrieben. Es zeigen:
- Figuren 1 bis 3:: schematische, vergrößerte Schnittansichten von Ausführungsformen erfindungsgemäßer Generatoreinrichtungen;
- Figuren 4 und 5:: eine schematische Schnittansicht und eine Perspektivansicht einer weiteren Ausführungsform der erfindungsgemäßen Generatoreinrichtung;
- Figur 6A:: eine Darstellung einer strukturierten Oberfläche, die als Substrat zur Herstellung der Generatoreinrichtung verwendet werden kann;
- Figur 6B:: eine photografische Darstellung eines Elektroden-Gegenelektroden-Paares, zum Beispiel bei einer Elektrodeneinrichtung gemäß Figur 5;
- Figur 7:: eine schematische Schnittansicht einer weiteren Ausführungsform der erfindungsgemäßen Generatoreinrichtung mit einer Kultivierungseinrichtung; und
- Figur 8:: eine schematische Illustration einer herkömmlichen elektrophysiologischen Messung des Membranpotentials.

Ausführungsformen der Erfindung werden im Folgenden in Bezug auf bevorzugte Merkmale der Elektrodeneinrichtung, der Generatoreinrichtung und des Verfahrens zu deren Betrieb erläutert. Zellbiologische und elektrochemische Gesichtspunkte, wie zum Beispiel die Erzeugung des Membranpotentials, die Bereitstellung von Kultivierungsbedingungen für adhärente Zellen, die Integration von Ionenpumpen (und -kanälen) bildenden Proteinen in Lipid-Membranen, werden hier nicht beschrieben, da sie an sich aus der Zellbiologie und der Elektrochemie bekannt sind. Es wird betont, dass die Umsetzung der Erfindung nicht auf die beispielhaft genannten Typen biologischer oder synthetischer Zellen beschränkt, sondern entsprechend mit allen membranumhüllten Zellen möglich ist, die zur Bildung eines Membranpotentials durch einen Transport geladener Teilchen, insbesondere Ionen, durch die Membran geeignet sind.

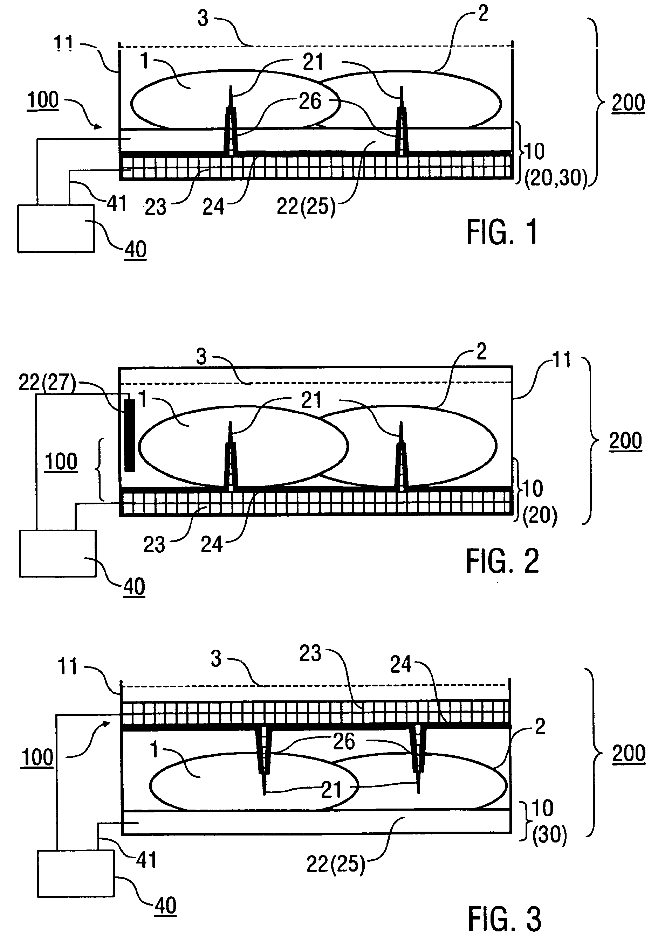

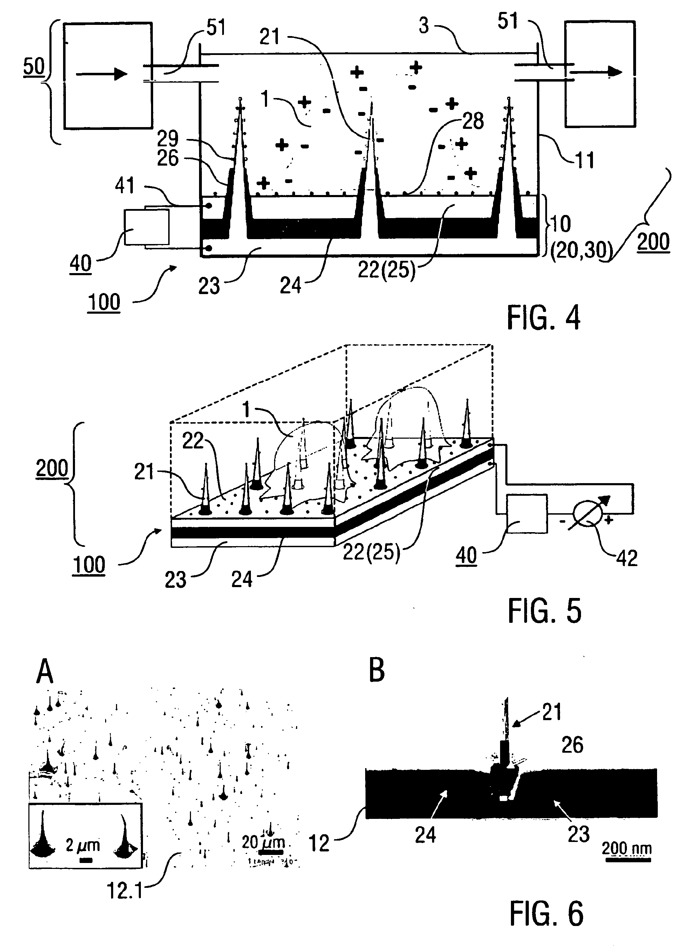

Ausführungsformen der Erfindung werden unter beispielhaftem Bezug auf die Stromerzeugung mit zwei Zellen (Figuren 1 bis 3) oder mit mehr Zellen (Figuren 3, 4) beschrieben. Es wird betont, dass die Umsetzung der Erfindung nicht auf die beschriebenen Beispiele beschränkt ist. Die Gestaltung der erfindungsgemäßen Generatoreinrichtung, insbesondere deren Grö-βe und die Einrichtungen zur Versorgung der Zellen, können in Abhängigkeit von den konkreten Bedingungen der Anwendung der Erfindung gewählt und angepasst werden.

Im Folgenden wird beispielhaft auf Ausführungsformen der Erfindung Bezug genommen, bei denen die Elektroden auf der Oberfläche des Elektrodenträgers Kathode sind und die mindestens eine Gegenelektrode eine Anode ist. Mit dieser Zuordnung der Polaritäten erfolgt die Stromerzeugung durch Ableitung von Membranpotentialen, die durch den Transport von Ionen in den und/oder aus dem Innenraum der Zelle gebildet werden. Das Membranpotential wird z. B. gebildet, indem eine Na⁺-K⁺-Pumpe für drei Natriumionen, die aus der Zelle gepumpt werden, nur zwei Kaliumionen in die Zelle pumpt. Die Umsetzung der Erfindung ist entsprechend mit umgekehrter Polarität möglich, indem die Elektroden Kathode und die mindestens eine Gegenelektrode mindestens eine Anode darstellen.

Gemäß Figur 1 umfasst die Generatoreinrichtung 200 eine Elektrodeneinrichtung 100 mit einer Zellaufnahme 10, die gleichzeitig den Elektrodenträger 20 und den Gegenelektrodenträger 30 bildet. Der Elektrodenträger 20 umfasst als Elektroden Kathoden 21 und als Gegenelektrode eine Anode 22, auf deren Oberfläche Zellen 1 angeordnet sind. Die Elektrodeneinrichtung 100 ist mit den Zellen 1 in einem Generatorgefäß 11 angeordnet, das ein Ionen-enthaltendes Medium 3 enthält und mit einer Kultivierungseinrichtung (in Figur 1 nicht dargestellt) verbunden sein kann.

Der Elektrodenträger 20 umfasst einen Mehrschichtaufbau aus einer unteren Kathodenschicht 23, einer mittleren Isolatorschicht 24 und einer oberen Anodenschicht 25, die zugleich die Anode 22 und die Oberfläche zur Aufnahme der Zellen 1 in einem adhärenten Zustand bildet. Die Außenseite der Membranhülle 2 der Zelle 1 hat einen direkten elektrischen Kontakt mit der Anode 23. Die Kathodenschicht 23 und die Anodenschicht 25 sind über elektrische Leitungen 41 mit einem Verbraucher-Stromkreis 40 und/oder einer Akkumulatoreinrichtung (schematisch dargestellt) verbunden. Die Herstellung des Schichtaufbaus aus der Kathodenschicht 23, der Isolatorschicht 24 und der Anodenschicht 25 wird mit weiteren Einzelheiten unten unter Bezug auf die Figuren 4 und 5 beschrieben.

Die Kathoden 21 ragen als spitzenförmige Vorsprünge von der Kathodenschicht 23 durch die Anodenschicht 25 über deren Oberfläche in das Generatorgefäß 11. Zur elektrischen Isolation der Kathoden 21 relativ zur Anode 23 bildet die Isolatorschicht 24 an jeder Kathode 21 einen Isolatormantel 26, der die Kathode 21 umgibt. Der Isolatormantel 26 erstreckt sich in Längsrichtung der Kathode 21 mit einer Länge, die mindestens gleich der Dicke der Anodenschicht 25 ist. Vorzugsweise erstreckt sich der Isolatormantel 26, wie in Figur 1 dargestellt, über die Oberfläche der Anodenschicht 25 in das Generatorgefäß 11, um die elektrische Isolation der Kathode 21 und der Anode 22 zu verbessern. Die axiale Länge des Isolatormantels 26 ist so gewählt, dass ein freies Ende der Kathode 21 (Kathodenspitze) im Generatorgefäß 11 freiliegt.

Allgemein umfassen die Kathoden 21 vorzugsweise Kohlenstoff, ein inertes Metall, wie z. B. Gold, oder einen dotierten Halbleiter. Die Isolatorschicht 24 wird durch eine Keramikschicht, wie zum Beispiel ein Metalloxid, gebildet. Die Anode 22 besteht ebenfalls vorzugsweise aus Kohlenstoff, einem inerten Metall oder einem dotierten Halbleiter.

Die Zellen 1 mit den Membranhüllen 2 sind so auf der Oberfläche der Anodenschicht 25 angeordnet, dass die Kathoden 21 mit dem Isolatormantel 26 in den Innenraum der Zellen 1 ragen. Jede Kathodenspitze ist in direktem elektrischen Kontakt mit dem inneren Zellmaterial, wie zum Beispiel im Falle einer biologischen Zelle mit dem Zellplasma oder im Fall einer synthetischen Zelle mit einem Innenmedium einer Vesikel. Die Zellen 1 umfassen zum Beispiel lebende biologische Zellen, wie zum Beispiel Epithelzellen oder Fibroblasten. Das Ionen-enthaltende Medium 3 im Generatorgefäß 11 umfasst ein Kultivierungsmedium, wie zum Beispiel eine physiologische Salzlösung, aus der Ionen durch die Membranhüllen 2 in das Innere der Zellen 1 transportiert werden. Das dabei gebildete Membranpotential wird über die elektrischen Leitungen 41 abgeleitet. Sobald die Ionenpumpen in der Membranhüllen 2 einen Ladungsausgleich zwischen dem Innenraum der Zellen 1 und dem äußeren Medium erfassen, erfolgt ein weiterer Ionentransport in den Innenraum der Zellen 1.

Biologische Zellen, die zur Stromerzeugung verwendet werden können, umfassen zum Beispiel kardiale Myozyten (HL1), Epithelzellen oder Fibroblasten (z.B. REF-52). Fibroblasten haben den besonderen Vorteil, dass sie stabile, langlebige und sich selbst reparierende, geschlossene Monoschichten auf festen Oberflächen, insbesondere auf der Oberfläche der Anodenschicht 25 oder einer Isolatorschicht 24 (siehe unten) bilden.

Vorteilhafterweise können als biologische Zellen NRK-Zellen ("normal rat kidney"-Zellen, insbesondere NRK-Fibroblasten) verwendet werden. NRK-Fibroblasten sind beispielsweise in der Publikation von J. J. Torres in "Am. J. Physiol. Cell Physiol." (Bd. 287, 2004, S. C851 - C865) beschrieben. NRK-Zellen bilden stabile Monolagen aus, wobei die Einzelzellen über Membrankanäle (so genannte "gap junctions") verbunden sind (siehe Fig. 1C der genannten Publikation). Die Membrankanäle sorgen für eine effiziente elektrische Verschaltung der Zellen und damit für eine deutliche Steigerung der Kapazität der erfindungsgemäßen Generatoreinrichtung, insbesondere in Abhängigkeit von der Elektrodendichte. Bei gleichzeitiger Erniedrigung der Elektrodendichte, die zu einer höheren Überlebensrate der Zellen führen kann, werden gleich bleibend hohe oder sogar gesteigerte Kapazitäten realisiert, da auch nicht direkt angestochene Zellen an das System angekoppelt werden.

Die biologischen Zellen können mit an sich bekannten biomolekularen Verfahren manipuliert sein, um zum Beispiel stabile Zellklone zu bilden, die sich durch eine Überexpression von bestimmten Ionenkanälen und -pumpen auszeichnen, um die elektrische Leistungsfähigkeit zu erhöhen. Zum Beispiel kann durch Überexpression von Connexin-Molekülen (Bausteine der obigen gap junction - Kanäle) - analog zur Expression von Ionenkanälen/Pumpen im Hinblick auf das Membranpotential - die elektrische Kopplung/Kapazität in beliebigen Zellen oder auch Vesikeln künstlich erhöht werden.

Synthetische Vesikeln mit einfachen oder mehrschichtigen Membranhüllen können mit an sich bekannten Standardtechniken hergestellt werden (siehe zum Beispiel A. Moscho et al. in "Proc. Natl. Acad. Sci.", Bd. 93, 1996, S. 11443 - 11447). Es können Vesikeln mit einem Durchmesser z. B. im Bereich von 1 µm bis 50 µm oder oberhalb 50 µm vorgesehen sein (sog. "giant vesicles", Riesenvesikeln). Die Selbstintegration der Zellen auf der Elektrodeneinrichtung wird durch die Biofunktionalisierung der Elektrodenoberflächen mit einer Adhäsionssteigern-den Beschichtung und/oder einer Endozytoseaktivierenden Beschichtung unterstützt.

In Figur 2 ist eine abgewandelte Ausführungsform der Generatoreinrichtung 200 schematisch illustriert, bei welcher die Zellaufnahme 10 bzw. der Elektrodenträger 20 der Elektrodeneinrichtung 100 lediglich die Kathodenschicht 23 mit den Kathoden 21 und die Isolatorschicht 24 aufweist. Als Gegenelektrode (Anode) ist ein in das Medium 3 im Generatorgefäß 11 getauchter Elektrodenstab 27 vorgesehen.

In Figur 3 ist eine weitere abgewandelte Ausführungsform der Generatoreinrichtung 200 schematisch illustriert, bei welcher die Zellaufnahme 10 die Anodenschicht 25 mit der Anode 22 umfasst, während der Elektrodenträger 20 mit den Kathoden 21 mit einem Abstand von der Zellaufnahme 10 angeordnet ist. Der Elektrodenträger 20 ist im Medium 3 im Generatorgefäß 11 so angeordnet, dass die von dem Elektrodenträger 20 hin zur Zellaufnahme 10 vorstehenden Kathoden 21 in das Innere der auf der Zellaufnahme 10 angeordneten Zellen 1 ragt.

Der Elektrodenträger 20 umfasst die Kathodenschicht 23 und die Isolatorschicht 24 mit dem Isolatormantel 26 für jede Kathode 21. Durch die Isolatorschicht 24 und die Isolatormäntel 26 sind die Kathoden 21 und die Kathodenschicht 23 gegenüber dem Medium 3 im Generatorgefäß 11 und der Anode 22 elektrisch isoliert. Die Anodenschicht 25 und die Kathodenschicht 23 sind über elektrische Leitungen 41 mit dem Verbraucher-Stromkreis 40 und/oder der Akkumulatoreinrichtung verbunden.

Die Ausführungsformen der Figuren 1 bis 3 können wie folgt weiter abgewandelt werden. Erstens können mehr als zwei Kathoden vorgesehen sein (siehe unten, Figuren 4, 5). Des Weiteren können die Varianten der Figuren 1 bis 3 kombiniert werden, indem Kathoden in zwei getrennte Elektrodenträger integriert sind und von zwei Seiten in die Zellen ragen. Der Mehrschichtaufbau aus den Kathoden-, Isolator- und Anodenschichten kann weitere Schichten aufweisen, z. B. eine Trägerschicht, mit der der Mehrschichtaufbau mechanisch stabilisiert wird und die z. B. aus Kunststoff gebildet ist (siehe Figur 6B).

Die Figuren 4 und 5 zeigen eine abgewandelte Ausführungsform der Erfindung, bei der ein Array von Kathoden vorgesehen ist, in schematischer Schnittansicht (Figur 4, Ausschnitt) und in schematischer Perspektivansicht (Figur 5). Diese Ausführungsform ist besonders für die Stromerzeugung durch Ableitung des Membranpotentials an Zellschichten, insbesondere einer Monoschicht, geeignet. Aus Übersichtlichkeitsgründen sind in den Figuren 4 und 5 jedoch lediglich einzelnen Zellen gezeigt.

Bei der Ausführungsform der Figuren 4 und 5 umfasst der Elektrodenträger 20, wie oben unter Bezug auf Figur 1 erläutert, einen Mehrschichtaufbau aus der Kathodenschicht 23, der Isolatorschicht 24 und der Anodenschicht 25. Die Kathodenschicht 23 und die Anodenschicht 25 sind über elektrische Leitungen 41 mit dem Verbraucher-Stromkreis 40 und/oder der Akkumulatoreinrichtung verbunden. Es kann eine Strommesseinrichtung 42 vorgesehen sein, die beispielhaft in Figur 5 gezeigt ist.

Die Kathoden 21 ragen, jeweils mit einem Isolatormantel 26 ummantelt, durch die Anodenschicht 25 in das Generatorgefäß 11. Das Generatorgefäß 11 ist mit einem Ionen-enthaltenden Medium 3 gefüllt, das von einer Kultivierungseinrichtung 50 (schematisch gezeigt) über Flüssigkeitsleitungen 51 zu- und abgeführt wird. Die Kultivierungseinrichtung 50 kann des weiteren eine Temperierungseinrichtung (nicht dargestellt) aufweisen, mit der die Temperatur der Generatoreinrichtung, z. B. auf eine physiologische Temperatur der Zellen, einstellbar ist.

Auf der Oberfläche der Anodenschicht 25 ist eine Adhäsionssteigernde (und die Leitfähigkeit steigernde) Beschichtung 28 vorgesehen, die zum Beispiel Fibronektin, Laminin, Peptide, zum Beispiel RDG-Sequenzen, oder Kollagen umfasst. Die freiliegenden Spitzen der Kathoden 21 tragen eine Endozytose-aktivierende Beschichtung 29, die zum Beispiel Moleküle umfasst, mit denen Membran-Bindungskräfte der Zelle lokal veränderlich sind, wie zum Beispiel Lektine oder SNARE-Proteine (Soluble N-ethylmaleimide-sensitive-factor Attachment Receptor-Protein).

Die Größe der Oberfläche der Anodenschicht 25 ist in Abhängigkeit von der gewünschten Leistung der Generatoreinrichtung 200 und somit der gewünschten Anzahl von Zellen 1, die zur Stromerzeugung beitragen, gewählt. Erfindungsgemäß kann die Elektrodeneinrichtung 100 zum Beispiel zur Aufnahme von mehr als 10, 100, 1.000, 10.000 oder mehr Zellen vorgesehen sein. Entsprechend kann die Zahl der Kathoden 21 10, 100, 1.000, 10.000 oder mehr betragen. Besonders bevorzugt weist die Fläche der Anodenschicht 25 eine Größe von mindestens 1 cm² auf.

Mit einem Zelldurchmesser von ungefähr 30 µm, wie zum Beispiel bei Fibroblasten-Zellen, können somit 100.000 Zellen oder mehr und ggf. 100.000 oder mehr Kathoden vorgesehen sein. Theoretische Abschätzungen ergeben für die Verwendung biologischer Zellen eine Gesamtleistung von rund 10 µW pro cm², wobei mit synthetischen Vesikeln höhere Leistungen, wie zum Beispiel 10 mW erreichbar sind.

Die Herstellung der Elektrodeneinrichtung 100 erfolgt nach einem Verfahren, mit dem Elektroden mit einer Länge von einigen Mikrometern und einem Durchmesser unterhalb von 100 nm hergestellt werden können. Erstens ist es möglich, die Kathoden 21 als Top-down-Prozess durch herkömmliche Lithographie und reaktives Ionenätzen (Deep Reactive Ion Etching, DRIE) herzustellen (siehe C. Greiner et al. in "Langmuir", Band 23, 2007, S. 3495-3502). Mit diesem Verfahren können Kathodenlängen von einigen Mikrometern erreicht werden. Bei Verwendung optischer Lithographie sind die Kathodendurchmesser auf einige 100 nm beschränkt. Kleinere Strukturen können erzeugt werden, wenn das DRIE-Verfahren mit einer Elektronenstrahl-Strukturierung oder einer Nanosphären- oder Block-Copolymer-Mizellen-Lithographie kombiniert wird.

Gemäß einer alternativen Variante können die Kathoden durch Abscheidungstechniken aufgebaut werden (Bottom-up-Prozess), wobei eine Flüssigkeits-Feststoff-Abscheidung (VLS-Verfahren) oder eine Metallhalogenid-Reduktion (MHR-Verfahren) vorgesehen ist. Im letzteren Fall werden metallische Keime, die auf einem Silizium-Wafer eine eutektische Schicht bilden, zum Wachstum von Silizium-Nanodrähten durch physikalische Dampfabscheidung verwendet (siehe L. Schubert et al. in "Applied Physics Letters", Band 84, 2004, S. 4968-4970). Im zweiten Fall können die Kathoden unter Verwendung von Metallhalogeniden als Keime gebildet werden (siehe P. Yang et al. in "JACS", Band 129, 2007, S. 7228-7229).

Gemäß einer weiteren Alternative können die Kathoden 21 durch Kohlenstoff-Nanoröhrchen gebildet werden, die in einer kohlenstoffreichen Atmosphäre aus Nickel- oder Eisen-basierten Keimen wachsen (siehe C. J. Lee et al. in "Chemical Physics Letters", Band 323, 2000, S. 554-559).

Die Elektrodeneinrichtung kann auch durch Anwendung eines Strukturierungsverfahrens hergestellt werden, das in WO 2007/096082 A1 beschrieben ist. Dabei ist vorzugsweise das folgende Mehrschritt-Verfahren vorgesehen. In einem ersten Schritt wird eine Oberfläche eines Polymer-Materials mit einer regelmäßig oder unregelmäßig verteilten Faden-Anordnung durch ein Schmelzen und anschließendes Ziehen der Fäden mittels einer Walze erzeugt, wie es in WO 2007/096082 A1 beschrieben ist. Diese Veröffentlichung wird hiermit durch Bezugnahme in die vorliegende Beschreibung einbezogen. In Figur 6A ist ein Formstück (Matrize) für die Herstellung der Elektrodeneinrichtung mit einer derart strukturierten Polymer-Oberfläche 12.1 beispielhaft gezeigt. In einem weiteren Schritt wird die strukturierte Oberfläche 12.1 dann mit Schichten aus Gold und SiO₂ und/oder Si₃N₄ beschichtet und strukturiert, um die hier beschriebene Schicht- und Elektrodenstruktur zu bilden. Die Beschichtung umfasst zum Beispiel eine physikalische Dampfabscheidung. Die Dicke der Schichten wird in Abhängigkeit von den konkreten Anwendungsbedingungen, insbesondere unter Berücksichtigung der elektrischen Leitfähigkeit der Gold-Schicht und den Isolationseigenschaften der SiO₂- und/oder Si₃N₄-Schicht gewählt. Anschließend wird zur Bereitstellung des elektrischen Kontakts zwischen dem Zytoplasma der eukariotischen Zellen und der leitfähigen Gold-Schicht die isolierende SiO₂- und/oder Si₃N₄-Schicht von denen Elektrodenspitzen durch nasschemisches Ätzen oder reaktives Ionenätzen entfernt. Schließlich folgt zur Optimierung des Eindringens der Elektroden in die Zellen und deren Fixierung und Lebensfähigkeit die Biofunktionalisierung der Oberfläche unter Verwendung von an sich bekannten Techniken.

Figur 6B zeigt eine photografische Darstellung eines Teils einer erfindungsgemäßen Elektrodeneinrichtung mit einer einzelnen Kathode 21 (graphisch markiert), die mit einem Scanning-Elektronen-Mikroskop (SEM) aufgenommen wurde. Der Schichtaufbau umfasst auf einer Trägerschicht 12 die Kathodenschicht 23 und die Anodenschicht 25 jeweils aus Gold, die durch die Isolatorschicht 24 aus SiO₂ getrennt sind. Die Kathoden 21 wachsen als Nanodrähte durch Löcher in der Anodenschicht 25, wobei die Kathodenschicht 23 als Keim verwendet wird. Die Kathode 21 erstreckt sich senkrecht zur Oberfläche der Anodenschicht 25.

Figur 7 illustriert weitere Einzelheiten eines Generatorgefä-βes 11, dass zur Aufnahme der erfindungsgemäßen Generatoreinrichtung 200 (schematisch ohne elektrische Leitungen illustriert) vorgesehen und mit der Kultivierungseinrichtung 50 (schematisch illustriert) verbunden ist. Die Generatoreinrichtung 200 kann im Generatorgefäß 11 positioniert und/oder in den Boden des Generatorgefäßes 11 integriert sein. Das Generatorgefäß 11 ist zwischen einem Unterteil 11.1 und einem Oberteil 11.2 gebildet. Das Unterteil 11.1 weist eine zylinderförmige Ausnehmung auf, in der die Generatoreinrichtung 200 eingeordnet ist. Das Oberteil 11.2 weist eine Ausnehmung auf, die an das Unterteil 11.1 angrenzend kegelförmig und zur Oberseite des Oberteils 11.2 hin zylinderförmig gebildet ist. Durch den kegelförmigen Bereich wird eine geneigte Begrenzung des Generatorgefäßes 11 gebildet, die für die Ableitung vom Luftblasen von Vorteil ist. Der zylinderförmige Bereich bildet einen Ansatz für einen Verschluss 11.3 des Generatorsgefäßes 11. Hierzu ist am Oberteil 11.2 ein Stutzen 11.4 mit einem Außengewinde vorgesehen, das mit einem Innengewinde am Verschluss 11.3 zusammenwirkt. Der Verschluss 11.3 enthält einen Dichtungsring 11.31 und eine Entlüftungsöffnung 11.32.

In dem Unterteil 11.1 sind Flüssigkeitsleitungen 51 zur Verbindung des Generatorgefäßes 11 mit den Teilen der Kultivierungseinrichtung 50 integriert. Die Flüssigkeitsleitungen 51 weisen Kupplungsbereiche 52 auf, die jeweils mit einem Ventil 53 zur Einstellung des Medienzuflusses und einem Keramikfilter 54 zur Filterung des Medienzuflusses ausgestattet sind. Die Oberseite des Unterteils 11.1 und die Unterseite des Oberteils 11.2 sind mit zu einander passenden Formen gebildet. Zwischen den Unter- und Oberteilen 11.1, 11.2 ist eine Dichtung 11.5 vorgesehen. Die Unter- und Oberteile 11.1, 11.2 sind über eine Verbindungseinrichtung 11.6 miteinander gekoppelt, die z.B. eine Schraub- oder Rastverbindung bildet. Vorteilhafterweise bildet die Verbindungseinrichtung 11.6 zugleich eine Führung für eine Stapelung für eine Vielzahl von Generatorgefäßen 11. So sind an der Oberseite des Oberteils 11.2 ein Vorsprung 11.7 und an der Unterseite des Unterteils 11.1 eine Ausnehmung 11.8 der Verbindungseinrichtung 11.6 vorgesehen, die zu einander komplementäre Formen aufweisen. Der Vorsprung 11.7 wird z. B. durch den Schraubenkopf einer Schraubverbindung gebildet.

Erfindungsgemäß verwendete Generatorgefäße sind vorzugsweise stapelbar. Im zusammengesetzten Zustand einer Vielzahl von Generatorgefäßen 11 sitzt ein oberes Generatorgefäß 11 auf einem unteren Generatorgefäß 11, so dass die Vorsprünge 11.7 des unteren Generatorgefäßes 11 in die Ausnehmungen 11.8 des oberen Generatorgefäßes 11 ragen. Alternativ zur Darstellung in Figur 7 können die Vorsprünge 11.7 und die Ausnehmungen 11.8 unabhängig von der Verbindungseinrichtung 11.6 auf den Oberflächen der Ober- und Unterteile 11.2, 11.1 gebildet sein.

Die in der vorstehenden Beschreibung, den Zeichnungen und den Ansprüchen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Elektrodeneinrichtung (100), die zur Membranpotential-Ableitung an Zellen (1) mit Membranhüllen (2) eingerichtet ist, umfassend:
(a) eine Zellaufnahme (10), die zur Fixierung der Zellen eingerichtet ist, und
(b) einen Elektrodenträger (20), der mindestens zwei Elektroden (21) einer ersten Polarität aufweist, wobei
(c) die Elektroden (21) als Vorsprünge gebildet sind, die über eine Oberfläche des Elektrodenträgers (20) ragen, und relativ zu der Oberfläche des Elektrodenträgers (20) elektrisch isoliert sind, und wobei
(d) die Elektroden (21) so angeordnet sind, dass wenn die Zellaufnahme (10) mit Zellen (1) versehen ist, die Elektroden (21) in den Zellen (1) positioniert sind
**dadurch gekennzeichnet, dass**
die Elektroden (21) und die mindestens eine Gegenelektrode (22) dazu eingerichtet sind, mit einem Verbraucher-Stromkreis (40) und/oder einer Akkumulatoreinrichtung (50) verbunden zu werden.

2. Elektrodeneinrichtung gemäß Anspruch 1, bei der
- die Elektroden (21) als Vorsprünge gebildet sind, die relativ zu der Oberfläche der Zellaufnahme (10) elektrisch isoliert sind, und/oder
- jede der Elektroden (21) einen Isolatormantel (26) aufweist, der die Elektrode (21) umgibt, sich in Längsrichtung der Elektrode (21) erstreckt und ein Ende der Elektrode (21) frei lässt.

3. Elektrodeneinrichtung gemäß einem der vorhergehenden Ansprüche, die umfasst:
- mindestens eine Gegenelektrode (22) einer zweiten, entgegengesetzten Polarität, die auf der Oberfläche des Elektrodenträgers (20) und/oder an einem Gegenelektrodenträger (30) gebildet ist, der einen Abstand von dem Elektrodenträger (20) aufweist.

4. Elektrodeneinrichtung gemäß einem der vorhergehenden Ansprüche, bei der
- der Elektrodenträger (20) einen Mehrschichtaufbau mit einer Elektrodenschicht (23), einer Isolatorschicht (24) und einer Gegenelektrodenschicht (25) aufweist, wobei die Elektroden (21) elektrisch isoliert von der Elektrodenschicht (23) durch die Isolatorschicht (24) und die Gegenelektrodenschicht (25) über die Oberfläche der Gegenelektrodenschicht (25) ragen,
- die Elektroden eine Endozytose-aktivierende Beschichtung tragen, und/oder
- die mindestens eine Gegenelektrode eine adhäsionssteigernde und/oder leitfähigkeitsteigernde Beschichtung trägt.

5. Elektrodeneinrichtung gemäß einem der vorhergehenden Ansprüche, bei der die Elektroden Mikroelektroden umfassen,
- deren Durchmesser geringer als 10 µm ist,
- deren Spitze einen Abstand von dem Elektrodenträger (20) aufweist, der mindestens 10 nm beträgt, und/oder
- die aus Kohlenstoff, einem inerten Metall oder einem dotierten Halbleiter gebildet sind.

6. Elektrodeneinrichtung gemäß einem der vorhergehenden Ansprüche, bei der
- mindestens zehn, insbesondere mindestens hundert elektrisch miteinander verbundene Elektroden (21) vorgesehen sind.

7. Elektrodeneinrichtung gemäß einem der vorhergehenden Ansprüche, wobei die Zellaufnahme (10) durch den Elektrodenträger (20) oder den Gegenelektrodenträger (30) gebildet ist.

8. Generatoreinrichtung (200), die zur Erzeugung elektrischen Stroms durch Membranpotential-Ableitung an Zellen (1) mit einer Membranhülle (2) eingerichtet ist, umfassend:
(a) eine Elektrodeneinrichtung (100) gemäß einem der vorhergehenden Ansprüche, und
(b) die Zellen (1), die an der Zellaufnahme (10) der Elektrodeneinrichtung (100) angeordnet sind.

9. Generatoreinrichtung gemäß Anspruch 8, bei der
- die Zellen (1) biologische Zellen oder synthetische Vesikeln umfassen.

10. Generatoreinrichtung gemäß einem der Ansprüche 8 oder 9, bei der
- die Zellen (1) an der Zellaufnahme (10) eine geschlossene Zellschicht bilden, und/oder
- die Zellen (1) mindestens eine Ionen-bindende Substanz enthalten.

11. Generatoreinrichtung gemäß einem der Ansprüche 8 bis 10, die umfasst:
- eine Kultivierungseinrichtung, die zur Zuführung eines Ionen enthaltenden Mediums zu den Zellen (1) eingerichtet ist,
- einen Verbraucher-Stromkreis (40), mit dem die Elektrodeneinrichtung (100) verbunden ist, und/oder
- eine Akkumulatoreinrichtung (50), mit der die Elektrodeneinrichtung (100) verbunden ist.

12. Verfahren zur Erzeugung von elektrischem Strom durch Ableitung eines Membranpotentials an Zellen (1) mit Membranhüllen (2), mit den Schritten:
(a) Bereitstellung einer Generatoreinrichtung (200) gemäß einem der Ansprüche 8 bis 11, und
(b) Ableitung eines elektrischen Stroms von der Elektrodeneinrichtung (100).

13. Verfahren gemäß Anspruch 12, mit dem Schritt:
- Zuführung eines Ionen-enthaltenden Mediums zu den Zellen (1), und/oder
- Kultivierung der Zellen (1) an der Zellaufnahme (10).

14. Verfahren gemäß Anspruch 12 oder 13, bei dem
- die Zellen (1) biologische Zellen oder synthetische Vesikeln umfassen, die sich durch eine Selbstintegration selbstständig mit den Elektroden kontaktieren.

15. Verwendung einer Elektrodeneinrichtung (100) oder einer Generatoreinrichtung (200) gemäß einem der Ansprüche 1 bis 7 zur Erzeugung von elektrischem Strom für einen Verbraucher-Stromkreis (40) und/oder eine Akkumulatoreinrichtung (50).

## Claims

1. An electrode device (100), which is adapted for deriving membrane potential at cells (1) with membrane envelopes (2), comprising:
(a) a cell holder (10) which is adapted for fixing the cells, and
(b) an electrode carrier (20) which has at least two electrodes (21) of a first polarity, wherein
(c) the electrodes (21) are formed as projections which project above a surface of the electrode carrier (20) and are electrically insulated relative to the surface of the electrode carrier (20), and wherein
(d) the electrodes (21) are arranged in such a manner that when the cell holder (10) is provided with cells (1), the electrodes (21) are positioned in the cells (1),
**characterized in that**
the electrodes (21) and the at least one counter electrode (22) are adapted to be connected to a load circuit (40) and/or a rechargeable battery device (50).

2. The electrode device according to claim 1, in which
- the electrodes (21) are formed as projections which are electrically insulated relative to the surface of the cell holder (10), and/or
- each of the electrodes (21) has an insulating shell (26) which encloses the electrode (21), extends in the longitudinal direction of the electrode (21) and leaves one end of the electrode (21) free.

3. The electrode device according to any one of the preceding claims, which comprises:
- at least one counter electrode (22) of a second opposing polarity, which is formed on the surface of the electrode carrier (20) and/or on a counter electrode carrier (30) which has a spacing from the electrode carrier (20).

4. The electrode device according to any one of the preceding claims, in which
- the electrode carrier (20) has a multiple-layer construction with an electrode layer (23), an insulating layer (24) and a counter electrode layer (25), wherein the electrodes (21) project in an electrically insulated manner from the electrode layer (23) through the insulating layer (24) and the counter electrode layer (25) above the surface of the counter electrode layer (25),
- the electrodes carry an endocytosis-activating coating, and/or
- the at least one counter electrode carries an adhesion-promoting and/or conductivity-increasing coating.

5. The electrode device according to any one of the preceding claims, in which the electrodes comprise microelectrodes
- the diameter of which is smaller than 10 µm,
- the tip of which has a spacing from the electrode carrier (20) which is at least 10 nm, and/or
- which are formed from carbon, an inert metal or a doped semiconductor.

6. The electrode device according to any one of the preceding claims, in which
- at least ten, particularly at least a hundred mutually electrically connected electrodes (21) are provided.

7. The electrode device according to any one of the preceding claims, wherein the cell holder (10) is formed by the electrode carrier (20) or the counter electrode carrier (30).

8. A generator device (200), which is adapted for generating electric current by means of the deriving of a membrane potential at cells (1) with a membrane envelope (2), comprising:
(a) an electrode device (100) according to any one of the preceding claims, and
(b) the cells (1), which are arranged on the cell holder (10) of the electrode device (100).

9. The generator device according to claim 8, in which
- the cells (1) comprise biological cells or synthetic vesicles.

10. The generator device according to any one of claims 8 or 9, in which
- the cells (1) form a closed cell layer on the cell holder (10), and/or
- the cells (1) contain at least one ion-binding substance.

11. The generator device according to any one of claims 8 to 10, which comprises:
- a cultivation device which is adapted for supplying an ion-containing medium to the cells (1),
- a load circuit (40), to which the electrode device (100) is connected, and/or
- a rechargeable battery device (50), to which the electrode device (100) is connected.

12. A method for the generation of electric current by means of the deriving of a membrane potential at cells (1) with membrane envelopes (2), with the steps:
(a) provision of a generator device (200) according to any one of claims 8 to 11, and
(b) deriving of an electric current from the electrode device (100).

13. Method according to claim 12, with the step:
- supply of an ion-containing medium to the cells (1), and/or
- cultivation of the cells (1) on the cell holder (10).

14. The method according to claim 12 or 13, in which
- the cells (1) comprise biological cells or synthetic vesicles which contact the electrodes independently by means of a self integration.

15. Use of an electrode device (100) or a generator device (200) according to any one of claims 1 to 7 for the generation of electric current for a load circuit (40) and/or a rechargeable battery device (50).

## Revendications

1. Ensemble à électrodes (100), prévu pour la dérivation du potentiel de membrane sur des cellules (1) à enveloppe membranaire (2) et comprenant :
(a) un logement de cellules (10), prévu pour la fixation des cellules, et
(b) un support d'électrodes (20) comportant au moins deux électrodes (21) avec une première polarité,
(c) les électrodes (21) étant formées comme électrodes en saillie se dressant au-dessus d'une surface du support d'électrodes (20), et étant isolées électriquement par rapport à la surface du support d'électrodes (20), et
(d) les électrodes (21) étant disposées de telle manière que lesdites électrodes (21) sont disposées dans les cellules (1) quand le logement de cellules (10) est pourvu de cellules (1),
**caractérisé**
**en ce que** les électrodes (21) et la ou les contre-électrodes (22) sont prévues pour être reliées à un circuit électrique récepteur (40) et/ou à un dispositif d'accumulateur (50).

2. Ensemble à électrodes selon la revendication 1, où
- les électrodes (21) sont formées comme électrodes en saillie isolées électriquement par rapport à la surface du logement de cellules (10), et/ou
- où chacune des électrodes (21) comporte une gaine d'isolateur (26) qui entoure l'électrode (21), s'étend dans la direction longitudinale de l'électrode (21) et laisse dégagée une extrémité de l'électrode (21).

3. Ensemble à électrodes selon l'une des revendications précédentes, comprenant :
- au moins une contre-électrode (22) d'une deuxième polarité opposée, formée sur la surface du support d'électrodes (20) et/ou sur un support de contre-électrodes (30) espacé du support d'électrodes (20).

4. Ensemble à électrodes selon l'une des revendications précédentes, où
- le support d'électrodes (20) présente une structure à plusieurs couches, avec une couche d'électrodes (23), une couche d'isolateur (24) et une couche de contre-électrodes (25), les électrodes (21), isolées électriquement de la couche d'électrodes (23) par la couche d'isolateur (24) et la couche de contre-électrodes (25), se dressant au-dessus de la surface de la couche de contre-électrodes (25),
- les électrodes supportent un revêtement activateur d'endocytose, et/ou
- la ou les contre-électrodes supportent un revêtement promoteur d'adhérence et/ou promoteur de conductivité.

5. Ensemble à électrodes selon l'une des revendications précédentes, où les électrodes comprennent des micro-électrodes,
- dont le diamètre est inférieur à 10 µm,
- dont le sommet est espacé d'au moins 10 nm du support d'électrodes (20), et/ou
- qui sont constituées de carbone, d'un métal inerte ou d'un semi-conducteur dopé.

6. Ensemble à électrodes selon l'une des revendications précédentes, où
- au moins dix, en particulier au moins cent électrodes (21) électriquement reliées entre elles sont prévues.

7. Ensemble à électrodes selon l'une des revendications précédentes, où le logement de cellules (10) est formé par le support d'électrodes (20) ou le support de contre-électrodes (30).

8. Dispositif générateur (200), prévu pour la génération de courant électrique par dérivation du potentiel de membrane sur des cellules (1) à enveloppe membranaire (2), comprenant :
(a) un ensemble à électrodes (100) selon l'une des revendications précédentes, et
(b) les cellules (1) disposées sur le logement de cellules (10) de l'ensemble à électrodes (100).

9. Dispositif générateur selon la revendication 8, où
- les cellules (1) comprennent des cellules biologiques ou des vésicules synthétiques.

10. Dispositif générateur selon la revendication 8 ou la revendication 9, où
- les cellules (1) forment une couche de cellules fermée sur le logement de cellules (10), et/ou
- où les cellules (1) contiennent au moins une substance de liaison ionique.

11. Dispositif générateur selon l'une des revendications 8 à 10, comprenant :
- un dispositif de culture, prévu pour l'amenée d'un fluide contenant des ions vers les cellules (1),
- un circuit électrique récepteur (40) auquel l'ensemble à électrodes (100) est relié, et/ou
- un dispositif d'accumulateur (50) auquel l'ensemble à électrodes (100) est relié.

12. Procédé de génération de courant électrique par dérivation du potentiel de membrane sur des cellules (1) à enveloppe membranaire (2), comprenant les étapes suivantes :
(a) préparation d'un dispositif générateur (200) selon l'une des revendications 8 à 11, et
(b) dérivation d'un courant électrique depuis l'ensemble à électrodes (100).

13. Procédé selon la revendication 12, comprenant l'étape :
- d'amenée d'un fluide contenant des ions vers les cellules (1), et/ou
- de culture des cellules (1) sur le logement de cellules (10).

14. Procédé selon la revendication 12 ou la revendication 13, où
- les cellules (1) comprennent des cellules biologiques ou des vésicules synthétiques, qui entrent en contact spontanément avec les électrodes par auto-intégration.

15. Utilisation d'un ensemble à électrodes (100) ou d'un dispositif générateur (200) selon l'une des revendications 1 à 7 pour la génération de courant électrique pour un circuit électrique récepteur (40) et/ou un dispositif d'accumulateur (50).
